# EUROPEAN PATENT SPECIFICATION

(11) **EP 1 837 401 B1**
(45) Date of publication and mention of the grant of the patent: **17.03.2010**
(21) Application number: 04802470.7
(22) Date of filing: 15.12.2004
(51) Int. Cl.: C12N 15/57, C12N 9/74, C12N 15/81, A61P 7/04

(54) **A FIBRINOLYSIN OF AGKISTRODON ACUTUS VENOM AND ITS USAGE**
FIBRINOLYSIN AUS DEM GIFT VON AGKISTRODON ACUTUS UND DESSEN VERWENDUNG
FIBRINOLYSINE DE VENIN D'AGKISTRODON ACUTUS ET SON UTILISATION

(43) Date of publication of application: 26.09.2007
(73) Proprietor: Sun Yat-Sen University, Guangzhou, Guangdong 510089 (CN)
(72) Inventor: YAN, Guangmei, c/o Sun Yat-Sen University, Guangzhou, Guangdong 510089 (CN); CHEN, Jiashu, c/o Sun Yat-Sen University, Guangzhou, Guangdong 510089 (CN); QIU, Pengxin, c/o Sun Yat-Sen University, Guangzhou, Guangdong 510089 (CN); SHAN, Hong, c/o Sun Yat-Sen University, Guangzhou, Guangdong 510089 (CN)
(74) Representative: Kramer - Barske - Schmidtchen
(86) International application number: PCT/CN2004/001457
(87) International publication number: WO 2006/063486

(56) References cited:
- EP-A- 0 323 722
- EP-A1- 0 323 722
- CN-A- 1 181 421
- CN-A- 1 584 029
- DATABASE EMBL [Online] 16 March 2004 (2004-03-16), "Deinagkistrodon acutus acurhagin precursor mRNA, complete cds." XP002495053 retrieved from EBI accession no. EMBL:AY566610 Database accession no. AY566610
- WANG WEN-JENG ET AL: "Purification and characterization of a novel metalloproteinase, acurhagin, from Agkistrodon acutus venom." THROMBOSIS AND HAEMOSTASIS APR 2002, vol. 87, no. 4, April 2002 (2002-04), pages 641-650, XP002495052 ISSN: 0340-6245

## Description

### [Technology field]

This invention involves technology field of genetic engineering, which Specifically includes the fibrino(gen)lytic gene No:2 from Agikistrodon acutus, the gene vector, the genetic engineering host cell using the gene vector and the drugs prepared by the gene to counteract the thromboemolic disorders.

### [Technical background]

At present, thromboemolic disorders, especially myocardial infarction and apoplexy have become one of the main causes of mortality and morbidity. With the successful control over the most infectious diseases, the improvement of people living standard, the prevalent increase of people's life-span and the aging of population structure in China, the morbidity of cardiovascular and cerebrovascular thromboemolic disorders have ranked the first and second place among all the discuses. It is estimated that there are at least 300 million cases annually. It is reasonably expected that the morbidity of thromboemolic disorders should be higher in the future.

Nowadays, the drugs of thromboemolic disorders mainly include antiplatelet agents (i.e. aspirin), anticoagulant (i.e. heparin) and thrombolytic agents (ie. streptokinase and urokinase). Antiplatelet and anticoagulant therapy such as aspirin and heparin is thought to enhance the effects of thrombolysis by prevention of new fibrin formation. However they have no effect on existed thrombus. The clinically available thrombolytic agents, such as Streptokinase, urokinase and tissue plasminogen-activator(t-PA), are plasminogen activators and effectively dissolve the thrombus by activating plasminogen. That is to say, the common characterization of the thrombolytic agents is the indirect, slow and weak thrombolytic effect, especially when acting on the larger thrumbosis. On the condition when cardiovascular and cerebrovascular thrombosis occur, myocardium and neuron will die owing to hypoxia in few minutes after the blood stream is cut off, due to their indirect thrombolysis mechanism, the above three drugs have some side effects, such as less selectivity with thrombosis and hemorrhage. It limits their broad application that staphylokinase can induce anaphylaxis, and tissue plasminogen activator is also expensive.

It should be especially noted that thrombolytic agents are ineffective to 25% patients even though combinated with other agents. Moreover, reocclusion occurs in about 5%-30% of the initially successful cases treated with thrombolytic agents, which has no susceptive on the thrombolytic agents.

In conclusion, the thrombolytic agents can not completely meet the requirements of the clinical application. It is urgent to develop a more specific and efficient thrombolytic drugs.

Foreign studies on snake venom show that the venom of Agkistrodon contortrix and Crotalus atrox(western diamondback rattlesnake) contain directly-fibrinolytic enzymes without activating plasminogen. Which have showed thrombolytic activities in the venous thrombolysis model in rat. Additional when the thrombolytic activities were determined in vivo, hemorrhage and putrescence was not observed by microscopic examination for tissue sections from heart, liver, lung and kidney.

The thrombin-likc enzymes isolated from some snake venom used in Chinese clinics, such as ancord, ahylysantinfarctase, is to convert fibrinogen into fibrin derivative and lower plasma viscosity to achieve antithrombotic effect with the similar mechanism as thrombin. Their efficacy and mechanism can not be compared with thrombolytic agents at all.

The unique venom of Agikistrodon acutus in China contains components which can directly degrade fibrin/fibrinogen without activating plasminogen and have a strong biological activity. Several fibrinolytic proteins are obtained from 15 fractions using column chromatography purification methods, among which Fraction II possesses biological characteristics of high value: (1) Directly- thrombolysis: the fibrinolytic factors can degrade fibrin using the heated plate to inactivate plasminogen; (2) Quick action: In vitro experiments, the action of fibrinolytic factor are twice as fast us urokinase; (3) Highly efficiency: biological activity of fibrinolytic factor is higher than that of urokinase (130ug fibrinolytic factor is equivalent to 450U UK); (4) Low side effects: Although not-purified venom of Agikistrodon acutus has obvious side effect of bleeding, hemorrhage are not observed at the dosage of 500 ug/ml fibrinolytic factor. These strongly support fibrinolytic factor from the venom of Agikistrodon acutus may be an attractive agent for treating thromboemolic disorders.

However, there are regional and seasonal diversities in the composition and biological activity of the venom of Agikistrodon acutus from natural source, in fact it is difficult to achieve steady clinical effect; furthermore, owing to the complexity of crude snake components, it is still difficult to get single component even purification, which may lead to some toxicity and side effects in clinical application; Natural snake venom has the characteristics of limited production, high costs and low market occupancy. Its molecular structure and the relationship between structure and activity still can not be explained with protein technology. It is out of question to establish the scientific base of the clinical application of the fibrinolytic factor from the venom of Agikistrodon acutus through resolving these above issue.

Yeast can grow fast and be bulk fermentation without special culture medium, the yeast's DNA is relatively simple and convenient Cloning Screening. Yeast is fungus, so the mechanism of gene expression and regulation and processing modification of expression product are more completed than E. coli does. For example yeast can glycosylate amd form the correct disulfide bonds, etc. There have some precedents that snake venom protein successfully expressed in yeast. Using the antibody of FII from Agkistrodon acutus as probe, we screened the gene of FII from cDNA library and cloned to the yeast expression vector PPIC9K, then transformed into the yeast cell and achieved high expression level of FII from Agkistrodon acutus in yeast cell, at last we purified the recombinant FII from Agkistrodon acutus by means of Chromatography and determinated its fibrinolytic activity. Abundance recombinant FII from Agkistrodon acutus by genetic engineering were obtained in our study, which lay the foundation for the follow-up Pharmacodynamics study and the evaluation of the class 1 new drug.

Snake venom Researches have played a key role in the development of the biomedicine, such as isolation of DNA endonuclease from the snake venom which has promoted to the study of molecular biology greatly, the discovery of toxins from Coral snake which played a crucial role in the purification of neurotransmitter receptors. Compared with all kinds of chemical and biological clinical agents from snake venoms, recombinant FII from the venom of Agkistrodon acutus can overcome regional diversities and chemical inhomogeneity in the composition and biological activity, increase production and efficacy, become a new potential clinical drug with huge market value and own intellectual property rights.

DATABASE EMBL [Online] 16 March 2004, "Deinagkistrodon acutus acurhagin precursor mRNA, complete cds.", XP002495053 discloses a nucleic acid sequence encoding a Deinagkistrodon acutus acurhagin precursor.

W.-J. Wang et al., Thromb. Haemost. 2002, 87, 641-650 relates to the purification and characterization of the metalloproteinase acurhagin from Agkistrodon acutus venom.

EP 0 323 722 A1 discloses compositions and a method for the recombinant production of crotalidus venom fibrolase.

### [Invention]

fibrinolytic enzyme FII from Agkistrodon acutus can directly degrade fibrin, hemorrhage was not observed at the dosage of the effect fibrinolytic activity. However the components of crude snake venom arc complex and difficult to get a single component even purification. It isn't conducive to large-scale production because of the limited output of Snake venom. These above issue can be resolved through expressing recombinant fibrinolytic enzyme FII from Agkistrodon acutus by genetic engineering technology. We isolated FII, a fibrinolytic enzyme from the venom of Agkistrodon acutus, achieved the screening and cloning of the FII gene, then expressed the recombinant fibrinolytic enzyme in yeast cell, purified it and test its fibrinolytic activity.

Methods: the nature fibrinolytic enzyme, FII was isolated from Agkistrodon acutus venom by ion exchange chromatography and-gel filtration. Its fibrinolytic activity was determinated by Fibrin and fibrinogen used as substrate. Its reacting site on fibrinogen was observed by SDS-PAGE. Polyclonal antibody, monoclonal antibodies and cDNA library of Agkistrodon acutus venom were prepared. The gene of FII from cDNA library was screened by using the antibody of FII from Agkistrodon acutus as probe, which was cloned to the yeast expression vector PPIC9K by digestion and connection, yeast strains of high expression was obtained by transformation and screening. The recombination FII was purified by ion exchange chromatography and Hydrophobic chromatography. Its purity determination was tested by SDS-PAGE, fibrinolytic activity is determined by fibrin plate method, the effect on IgG and albumin was observed, and the fibrinolytic activity in vivo was determined by Beagle's pulmonary artery thrombosis model.

Result: with a three-step procedure, we obtained a fibrinolytic enzyme, FII, which appeared as a single band on SDS-PAGE and the molecular weight was 25,500. FII cleaved, primarily, the Λ α , B β chains of fibrinogen and fibrin, while the γ chain was minimally affected. FII degraded fibrin iri a dose-dependent manner. Through preparation of polyclonal antibody, monoclonal antibodies and cDNA library, we screened the gene FII from the cDNA library, constructed recombinant plasmid, PPIC9K-FII. After transformation, recombinant plasmid insert into yeast's chromosome determined by PCR. Using fibrinolytic activity as standard, we screened a high expression strain, and the fibrinolytic activity reached the peak on the third days of expression. With a two-step procedure, we obtained recombinant fibrinolytic enzyme FII, which appeared as a single band on SDS-PAGE. Recombinant FII can strongly degrade fibrin, while have no effect on IgG and albumin. Recombinant FII can efficiently dissolve the preformed thrombus in the right lower Pulmonary artery when measurements made at 1 hours post recombinant F II infusion. The average of the recanalization rate of thrombosis of the right lower Pulmonary artery was 83.3%. Conclusion: recombinant fibrinolytic enzyme FII was highly expressed in yeast, recombinant FII have high fibrinolytic activity.

Nature FII from Agkistrodon acutus snake venom have low yield, instable quality. We expressed recombinant FII in yeast by genetic engineering technology, the recombinant FII had higher fibrinolytic activity, more stable quality higher Output and lower cost than nature FII. The recombinant FII can dissolve effectively thrombus in animal thrombolysis models, and little hemorrhage was observed. It's a strong suggested that the recombinant FII will become a novel and effective thrombolytic agent which have tremendous benefits on Social and economic.
Fig1 : DEAE-SephadexΛ-50 ion exchange chromatograph of Agkistrodon acutus snake venom
Fig2: the first SephadexG-75 gel filtration of FII
Fig3: the second SephadexG-75 gel filtration of FIIa
Fig4: Determination of the molecular weight of FII by SDS-PAGE
Fig5: the Standard curve of Protein Markers using by SDS-PAGE.
Fig6: analysis of the degradation products of fibrinogen hydrolysis by SDS-PAGE.
Fig7: fibrinolytic activity of FII.
Fig8: analysis of the total RNA of Agkistrodon acutus venom by Agarose-Formaldehyde Electrophoresis
Fig9: the structure of cDNA library of Agkistrodon acutus venom
Fig10: the positive results of FII in the cDNA library of Agkistrodon acutus venom screened by monoclonal antibodies
Fig11: the nine Positive clones' sequences of inserting DNA fraction
Fig12: the amoid Sequence deduced by the DNA sequence of FII.

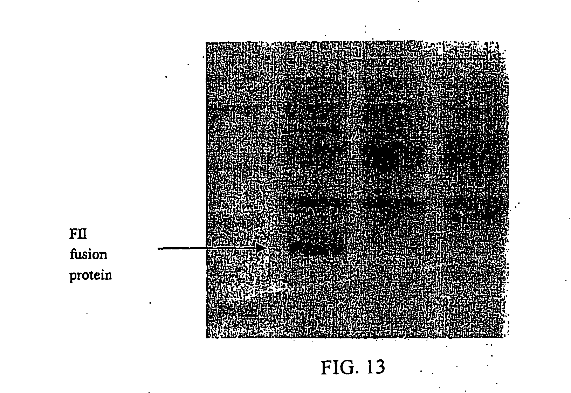

Fig13: analysis of FII fusion Protein expressing in E.coil, by SDS-PAGE.
Fig14: the two-step of constructing the target DNA-vctor of F II gene in E.coil.
Fig 15: the diploid restrictions in the plasmid Ppic9k-FII.
Fig16: fibrinolytic activity of FII in the supernatant by SDS-PAGE.
Fig17: DEAL-Sepharose icon exchange chromatography of the supernatant.
Fig 18: Buty-toyopcarl chromatography of the supernatant.
Fig19: determination of the recombinant FII purity by SDS-PAGE.
Fig20: directly fibrinolytic activity of recombinant FII.
Fig21: the effect on IgG and albumin of recombinant FII by SDS-PAGE.
Fig22: The effect of recombinant FII on the Beagle's pulmonary artery thrombosis model.

### Specific implementary scheme

### 1. Purification of FII from Agkistrodon acutus snake venom

### 1.1 DEAE-Sephadex A-50 ion exchange chromatography

Agkistrodon acutus snake venom(3g) was dissolved in 10ml 0.05M ammonium acetate(PH8.0), and the supernatant was applied to a column(3.0*80cm) of DEAE-Sephadex A-50 equilibrated with 0.05M ammonium acetate(PII8.0) at 25°C for 24h. Fractions were eluted with a linear gradient consisting of 0.05M ammonium acetate(PH 8.0) as starting buffer and 1M ammonium acetate(PH 5.0) as limit buffer. A volume of 3ml fraction was collected at a fow rate of 12ml/h. The absortance of eluate was tested at 280nm. The peak fractions tested fibrinolytic activity. Then the fibrinolytic fraction was dialyed and lyophilized.
fibrinolytic activity was demonstrated using a modified fibrin plate technique(Astrup and Mullertz, 1952). Each concentration was tested three times. Bovine plasma(0.2%,20ml) was clotted in a Petri dish(diameter, 9.5cm) with thrombin (80µl, 100U/ml). Fribrinolytic activity was expressed as the diameter product (mm²) of the lysed zone caused by 20ul of each fraction. Each fraction was put on the surface of the dish and incubated at 37°C for 12h.

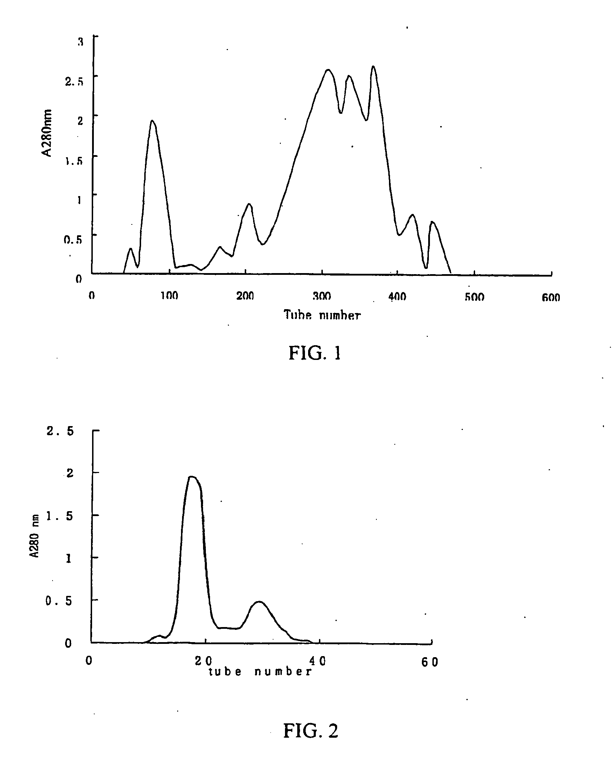

Results: Ion exchange chromatography of crude venom on DEAE-sephadex A-50 yielded ten fractions(Fig1). The second fraction (FII) has higher fibrinolytic activity. Its fibrinolytic activity was 60.23±16.47 mm²/µg.

### 1.2 The first sephadex G-75 gel filtration

FII (85mg) obtained from DEAF-Sephadex A-50 ion exchange chromatography was dissolved in 2ml 0.05M ammonium acetate(PF18.0), and the supernatant was applied to a column(1.1*100cm) of Sephadex G-75 equilibrated with 0.05M ummonium acetate(PII8.0) at 25°C for 24h. Fractions were eluted with 0.05M ammonium acetate(PH 8.0) A volume of 3ml fraction was collected at a low rate of 12ml/h. The absortance of eluate was tested at 280nm. The peak fractions tested fibrinolytic activity. Then the fibrinolytic fraction was dialyed and lyophilized.

Results: After further fractionation by gel filtration on a Sephadex G-75 column, two fractions of FII were obtained (Fig2). The fibrinolytic activity was localized in the first fraction. Its fibrinolytic activity was 87.51±14.95 mm²/µg.

### 1.3 The second sephadex G-75 gel filtration

The fibrinolytic fraction from the first Sephadex G-75 gel filtration was again applied to Sephadex G-75 get column by the above method.

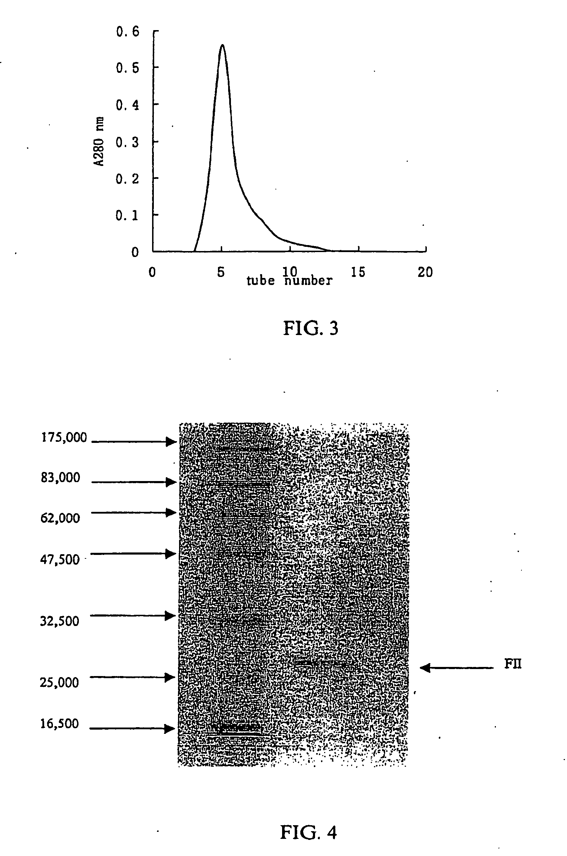

Results: The single peak was obtained (Fig3). Its fibrinolytic activity was 90.49±12.41 mm²/µg.

### 2. Purity and biochemical characterization determination

### 2.1 purity determination of the fibrinolytic enzyme FII from Agkistrodon acutus venom

Sodium dodecyl sulfate-polyacrylamide gel electrophoresis (SDS-PAGE) was performed according to the method of Laemmli (12% resolving gels, pH 8.8; 4% stacking gels, pH 6.8; Tris- glycine as the electrophoresis buffer composition; volve,200v; Time, 45min) Molecular weight standards were MBP-β-galactosidase (175,000), MBP-paramyosin (83,000), Glutamic dehydrogenase (62,000), Aldolase (47,500), Triosephosphate isomerase (32,500), β-Lactoglobulin A (25,000), Lysozyme (16,500). The protein bands were stained with 0.25% coomassie brilliant blue R-250 solution.

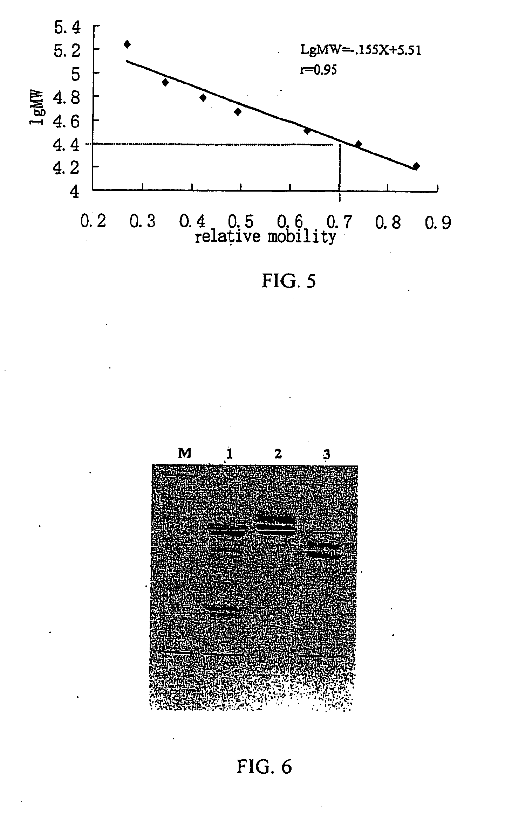

Results: On the SDS-PAGE gel, the purified FII appeared as a single protein band (Fig4). The equation, IgMW-1.55X15.51, r=0.9-5, was obtain according to the regression curve (X-axis, IgMW; Y-axis, the relative mobility of standard mobility(Rf(x))). Its calculated molecular weight is 25,550(Fig5). 2.2 fibrinogenolytic activity determination of the fibrinolytic enzyme FII from Agkistrodon acutus venom

Bovine fibrinogen (75µl, 0.2mg/ml) was pipetted into 1.5ml microcentrifuge tubes. FII (25µl, 2mg/ml) was added to the tube and the solution was incubated at 37°C for 1 hours. Then the reaction in the tube was stopped by the addition of 10µl of 125 Mm EDTA followed by 50µl of SDS-PAGE sample buffer. 20µl of sample was applied for SDS-PAGE analysis to determine whether FII degradation of degradation of fibrinogen varied from that of plasmin degradation, the same experiment was repeated except for plasmin in place of FII.

Resuls: SDS-PAGE showed FII is an α, β-fibrinogenase with the α, β chains being degraded, while the γ chain appeared unaffected. The MW or the degradation fraction by FII was 45,000Da. The α β and γ chains of fibrinogen were degraded by plasmin. The MW of the degradation fractions by plasmin was 47,00ODa, 44,00ODa, 23,000Da, respectively. The bands after degradation by FII and plasmin were different, which indicate they have different cleavage sites (Fig6).

### 2.3 fibrinolytic activity determination of fibrinolytic Enzyme FII from Agkistrodon acutus venom

Fibrinolytic activity was demonstrated using a modified fibrin plate technique (Astrup and Mullertz, 1952). Bovine Fibrinogen (0.2%,20ml) was clotted in a Petri dish(diameter, 9.5cm) with thrombin (80µl,100U/ml). Each concentration was tested three times. Fribrinolytic activity was expressed as the diameter product (mm²) of the lysed zone caused by 20ul of each fraction. Each fraction was put on the surface of the dish and incubated at 37°C for 12h. arokinase 500U/ml was used for positive control and the saline solution was used for the negative control.

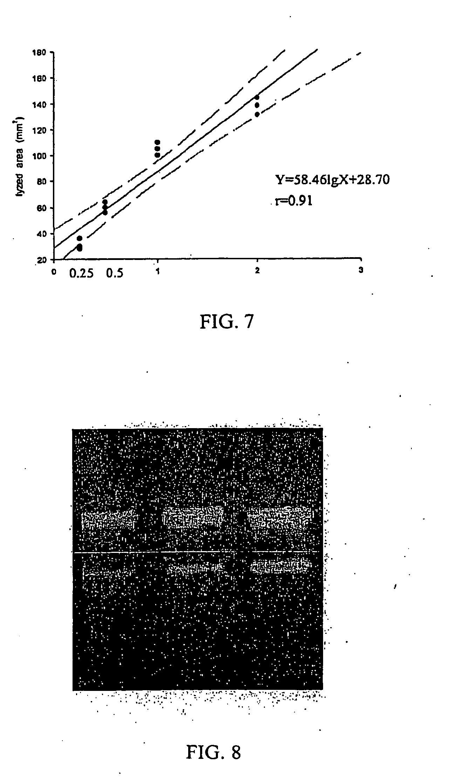

Results: FII, at 20ul at several different concentrations, 0.25,0.5, 1 and 2mg/ml was applied (table 1). It was shown that the lysed areas increased with the increased concentration (Fig7).

### 3. Preparation of the antibody of fibrinolytic enzyme FII from Agkistrodon acutus venom

### 3.1 Preparation of FII antiserum of rabbit

Method:
(1) Animal immunity. Rabbits were immunized by Multi-point Subcutaneous injection at the back with the Emulsifier mixed with 1ml of FII (3mg/ml) and 1ml Freund's adjuvant. After the first injection, the subsequent boosters were given per week, totally 4 week.
(2) Preparation of antiserum. The immunized rabbits were anaesthetized by an intramuscular injection of 30 mg/kg ketamine hydrochloride. Blood samples were taken through a catheter inserted into a Carotid and stored at 4 °C overnight. The sera were subsequently separated by centrifugation and aliquots were stored at 4 °C.
(3) Determinations of antibody titer by eiizymc-linked immunosorbent assay (ELISA). Microtiter plates (96 wells) were coated overnight at 4°C with 100 ml (0.1 mg/ml) of FII in sodium bicarbonate buffer, 100 µl per well and was stored at 4 °C overnight. The plates were washed two times with PBST containing 0.05% Tween-20 and unbound sites were blocked for 1h at 37 °C with blocked liquor containing 2 X PBS ,1% bovine serum albumin (BSA). To measure the serum titers, 100 ml of serial dilutions of serum in 1X PBS containing 1% BSA and a rabbit antiserum(primary antibody) were added to the plates and incubated for 1h at 37 °C. The plates were washed three times again and incubated for 1 h with 100 ml of a goat anti-rabbit immunoglobulin G (whole molecule)-peroxidase conjugate, followed by further washing for three times. The substrate solution for the peroxidase assay A and B were added into every well, the antibody titer was identified according to color of reaction system.

Result: NO.1 and 2 rabbits were immunized by the Purified fibrinolytic enzyme F 11 from Agkistrodon acutus venom using as antigen. After 4 weeks, antibody titer was determinated by ELISA. The antibody titer was 1:20000 in each rabbit.

### 3.2 Preparation of FII monoclone antibody

Method:
(1) immunity, hybridoma Fusion and cloning screening. Balb/c mice (7-week old) were subcutaneously injected with a mixture containing 0.2ml (1mg/ml)F II and an equal volume of Freund's complete adjuvant. A boost injection with the same amount of antigen in Freund's complete adjuvant was administered at 2-week intervals, totally 3 cycles. 0.2ml (1mg/ml) FII was injected through vena caudalis, 3 days later, hybridoma fusion was performed. In brief, the splenocytcs were harvested from immunized mice, mixed with SP2/10 cells at a 10:1 ratio, and fusion was carried out with 5θ% PEG(u 3700). The fused cells were cultured in 5 96-well plates, und cultured in a 5% CO2 incubator. HAT selective medium including 15% FBS were consecutively applied for 4 days, followed by HT medium for 6 days, from the eleventh day on RPMI-1640 medium was kept in using. Antibody produced in medium was measured by indirect enzyme-linked immunosorbent assay (indirect ELISA) as described above.
(2)Result: 2() cell lines were established after 2 cycles of hybridoma fusion. Among which, 2 cell lines with higher specificity were named C1 and C4, respectively. The monoclone antibody produced by the two cell lines all were Ig G 1, testing by Ig G typing kit, used according to the instructions of the manufacturer.

### 4. Construct an expression cDNA library from Agkistrodon acutus venom gland

### 4.1 Preparation of RNA

Method:
(1) The pairs of venom glands were extracted from the snakes' heads and were dissected and stored in solid carbon dioxide immediately.
(2) Using the RNA purification kit (Promega) and following its manufacturer protocol. The venom gland (for a total of about 1.7 g) was mixed. Trizol, chloroform and isopropyl alcohol were used respectively to homogenize the gland, centrifuge for 10 minutes at 12,000 rpm and separate the aqueous phase of RNA, deposit the total RNA with isopropyl alcohol. The RNA pellet was washed twice with 75% ethanol and briefly stir dried. In the end, the RNA was dissolved in diethylpyrocarbonate (DEPC) treated H2O. The integrity of total RNA was checked hy discerning the 28 S, 18 S and 5 S bands of ribosomal RNA in 1% agarose gel. The purity of the total RNA was checked by the ratio of A260/A280.

Result: the total RNA appeared as a long smear with clear bands of 28 S, 18 S and 5 S. the ratio of OD260/OD280 to the total RNA was 20, and the total amount wus 426ug. So we can draw the conclusion that the RNA didn't degrade and that the quality is high. RNA electrophoris (Fig.8) show 28 S and 18 S rRNA clearly and the fluorescence intensity of 28 S was two times that of 18 S rRNA, indicating a foundation for a high quality library.

### 4.2 purification of mRNA

Method: mRNA was subsequently purified by means of oligo (dT)-cellulose affinity chromatography, following its manufacturer protocol. Poly (A) RNA was enriched from total RNA: sample buffer solution sufficiently wash the cellulose column, the total RNA (3mg) in DEPC treated H2O was incubated at 65°C for 10 minutes. Decrease temperature to room temperature quickly by means of iced bath. Eluted with sample buffer solution for three times, followed by eluant, mRNA was washed and isolated.

The intactness of the RNA was assessed using 1% agarose ethidium bromide electrophoresis and The integrity of the mRNA was checked by 1 % agarose electrophoris.

Result: The total amount of mRNA was 10ug.

### 4.3 construction of a cDNA library

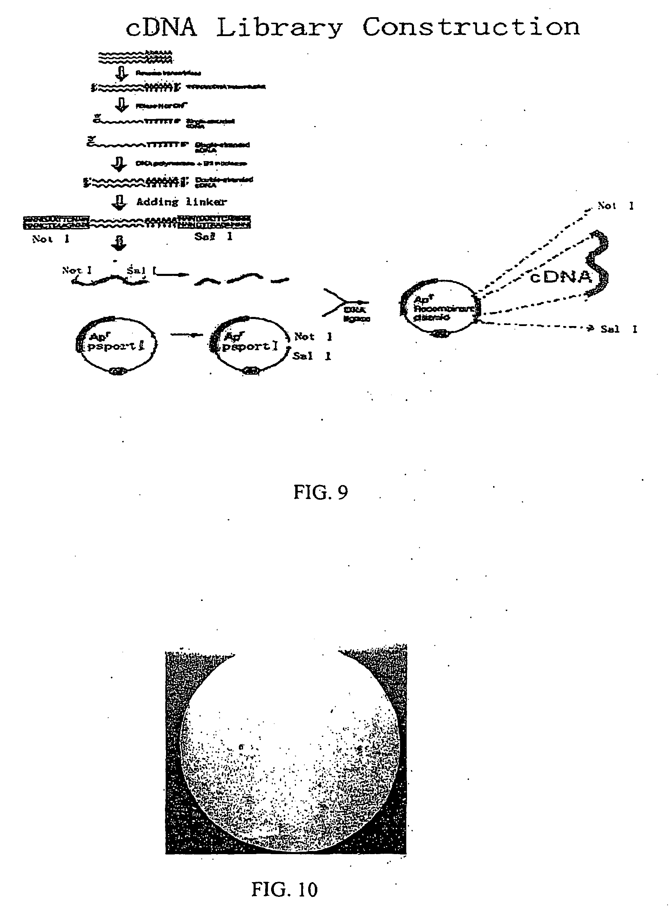

Method: using the cDNA synthesis kit (Promega) and following its manufacturer protocol. See (Fig.9)
(1) Synthesis of the first strand cDNA
(2) Synthesis of the second strand cDNA
(3) Adding Not and Sal 1 adaptors at the end of cDNA
(4) Ligation and transformation of cDNA cDNA and pSport 1 vector were ligated. The reaction mixtures of cDNA, pSport I vector, T4 ligase 10 × buffer, T4 DNA ligase were incubated at 16 °C overnight. 10 ul of product of ligation was added into 50 ul competence E coli DH5, in order, 4 °C, 30 min, 42 °C 120s, 4 °C, 5 min, 37 °C, 10min, Then the suspended cells were then placed into a new 1.5 mL EP tube and rotated at 37 °C for 1 h under 250 rpm. 2 µL of transformation product were diluted 10, 100 and 1000 times and plated on Luria-Bertani plates containing 100 mg L-1 of ampicillin with isopropy-β-D-thiogalactoside and 5-brom-4-chloro-3-indolyl-beta-D-galactopyranoside; Plates were incubated ut 37 °C overnight. The titer of the library was calculated and stored the library at -80 °C.

### Result: The total titers of five unamplified libraries are 5 x 107 pfu/ml.

### 5. Clone of F II gene from expression cDNA library

### 5.1 Screening expression cDNA library with antibodies

Method: after proper dilution, 100 µl bacterium in cDNA library were spreaded into plates containing LB medium supplemented with 1% glucose, 8% glycerol and 50ug/ml ampicillin and grown overnight at 37°C.

Cellulose acetate membrane was soaked with IPTG(10 mmol/l) and spreaded on the surface of J.B plates, three asymmetrical spot was left on the surface of cellulose acetate membrane to facilitate orientation. Unveil cellulose acetate membrane and spreaded on the surface of agarose plate, plates were incubated at 37 °C for 6-8h for induction of fusion protein expression.

After 6-8h of induction, cellulose acetate membrane was treated with chloroform for 30 min, the membrane were subsequently blocked overnight at 4 °C in 1% BSA , 4 ug/ml lysozyme and 1 ug/ml DNAase with gentle agitation for 30 min, After blocking, filters were washed three times for 15 min per wash in approximately 100 ml per filter of Tris buffered saline (TBS) with 0.2% Tween 20 and 0.05% Triton-X 100 (TBS-TT), followed by two washes in 100 ml/filter of TBS to remove residual detergent. To detect colonies producing recombinant protein, the membrane was probed with rabbit-antibody at u 1:1000 dilution in the blocking buffer with gentle agitation for 3 h at room temperature, The membrance were washed three times again and incubated for 1 h with 100 ml of a goat anti-rabbit immunoglobulin G (whole molecule)-peroxidase conjugate, followed by further washing for three times.

The membrane was soaked in DAB colouration solution with gentle agitation for 30 min, rinsed with distilled water, Positive colonies with brown color were detected.

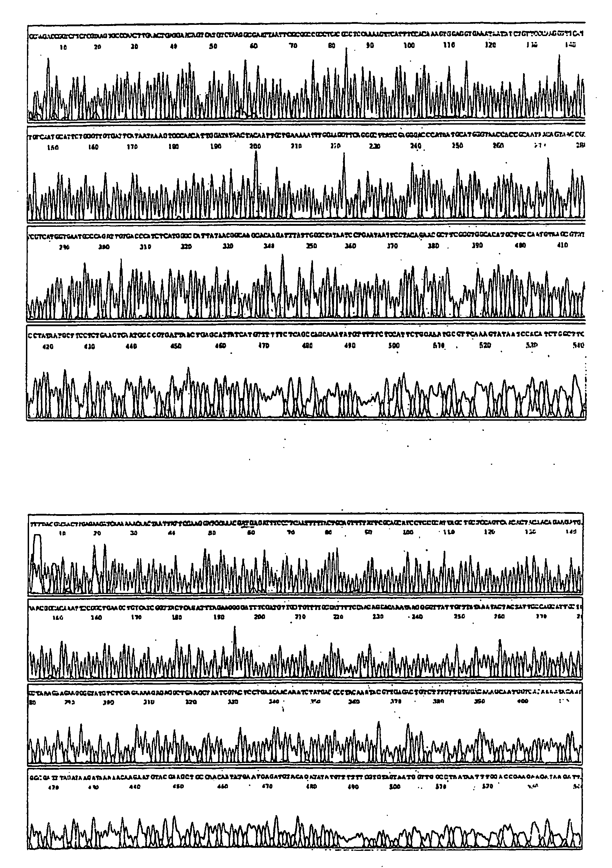

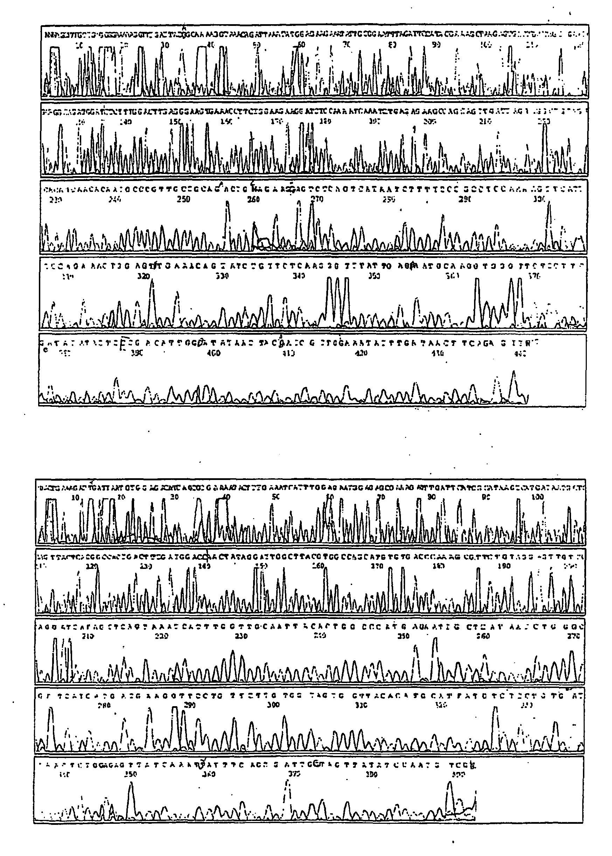

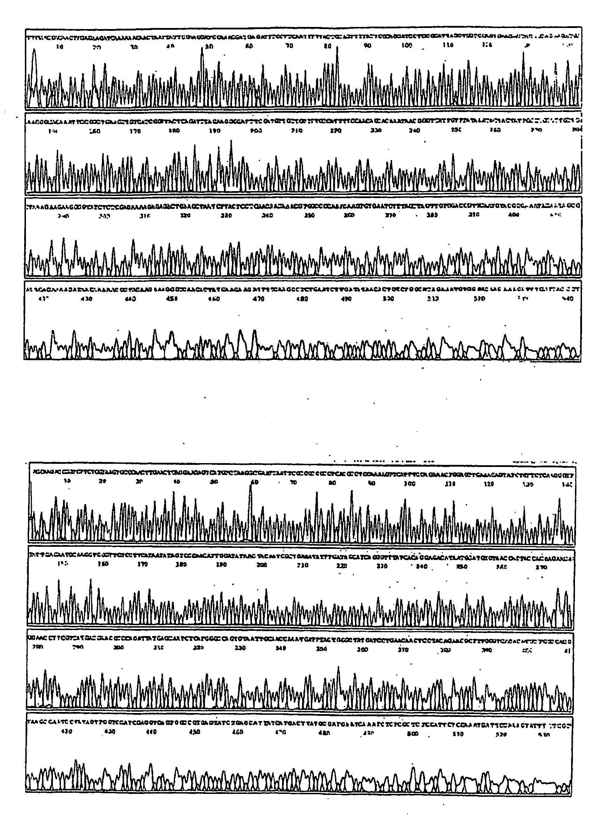

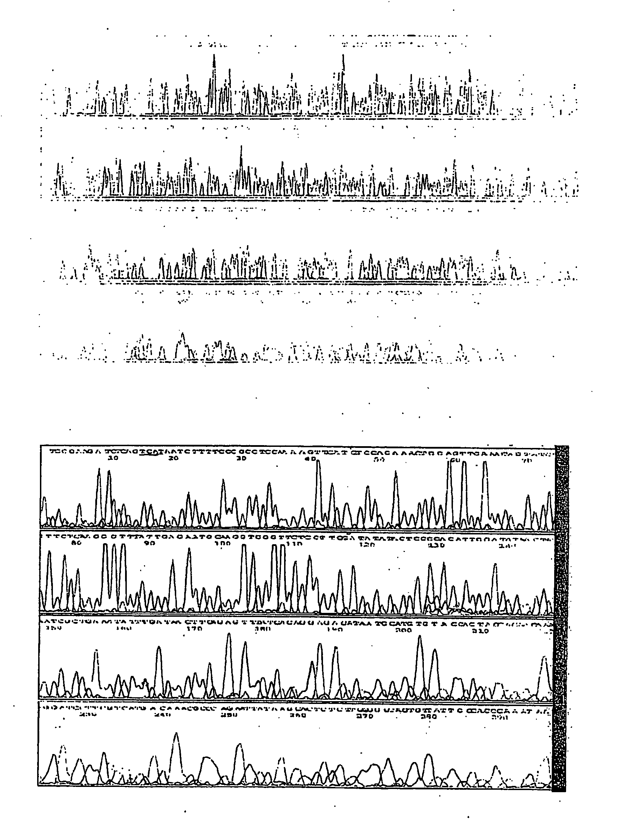

Results: primary screening was performed with platen (9 cm in diameter, including 5×103clones). 200,000 clones were screened and 15 positive colonies were found. Repeated screening were performed in those positive clones and positive reactions were observed in 11 clones (Fig. 10). Those 11 positive clones were plated on Luria-Bertani plates containing 100 mg·L-1 of ampicillin, detected with monoclone antibody, positive reactions (C1 and C4 antibody were used) were observed in 9 clones.

### 5.2 Plasmid extraction and sequencing of positive clone

Method: the plasmids were extracted by alkaline lysis (QIAGEN):
(1) Pick a single colony from a freshly streaked selective plate and inoculate a starter culture of 2-5 ml LB medium containing the appropriate selective antibiotic. Incubate for approximately 8 h at 37°C with vigorous shaking (approx. 300 rpm).
(2) Dilute the starter culture 1/500 to 1/1000 into 3 ml selective LB medium. Grow at 37°C for 12-16 h with vigorous shaking (upprox. 300 rpm).
(3) Harvest the bacterial cells by centrifugation at 6000 × g for 15 min at 4°C.
(4) Resuspend the bacterial pellet in 0.3 ml of Buffer P1.
(5) Add 0.3 ml of Buffer P2, mix thoroughly by vigorously inverting the sealed tube 4-6 times, and incubate at room temperature (15-25°C) for 5 min.
(6) Add 0.3 ml of chilled Buffer P3, mix immediately and thoroughly by vigorously inverting 4-6 times, and incubate on ice for 5 min.
(7) Centrifuge at maximum speed in a microcentrifuge for 10 min. Remove supernatant containing plasmid DNA promptly.
(8) Equilibrate a QlAGEN-tip 20 by applying 1 ml Buffer QRT, and allow the column to empty by gravity flow.
(9) Apply: the supernatant from step 7 to the QIAGEN-tip 20 and allow it to enter the resin by gravity flow.
(10) Wash the QIAGEN-tip 20 with 2 x 2 ml Buffer QC.
(11) Elute DNA with 0.8 ml Buffer QF. Collect the eluate in a 1.5 ml or 2 ml mier
(12) Precipitate DNA by adding 0.7 volumes (0.56 ml per 0.8 ml of elution volume) or room-temperature isopropanol to the cluted DNA. Mix and centrifuge immediately at > 10,000 rpm for 30 min in a microcentrifuge. Carefully decant the supernatant.
(13) Wash DNA pellet with 1 ml of 70% ethanol and centrifuge at 10,000 rpm for 10 min.
(14) Air-dry the pellet for 5-10 min, and redissolve the DNA in a suitable volume of buffer.

The plasmids were used for capillary sequencing applying the standard primers T7 and SP6.

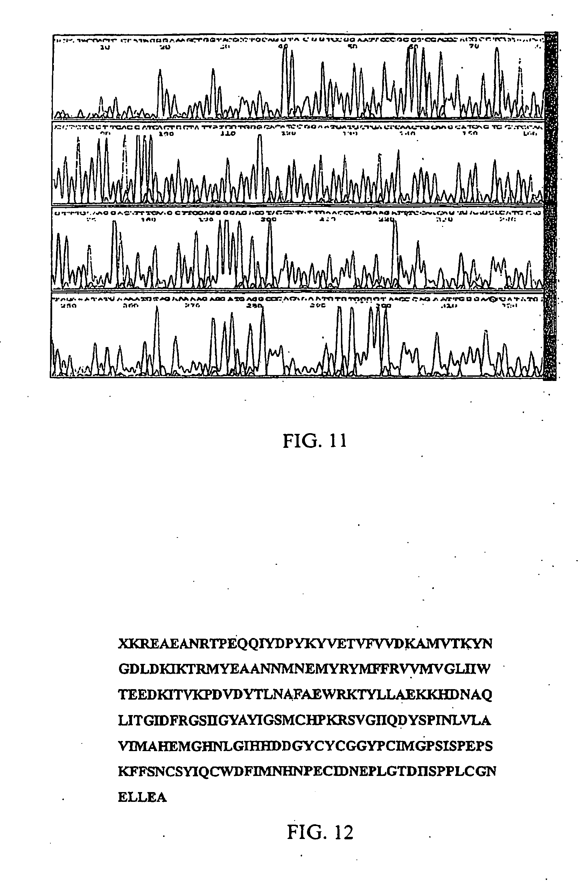

Result: Pick a single colony from the 9 positive plate respectively and inoculate a starter culture of 2-5 ml LB medium containing the appropriate selective antibiotic. Incubate for approximately 8 h at 37°C with vigorous shaking for extracting plasmid. PCR reaction was performed applying the standard primers T7 and SP6.specifical bonds of product were observed in each clone. The plasmids were used for capillary sequencing applying the standard primers T7 and SP6 (Fig.11).

### 5.3 Sequence alignment

Method: in order to identify the genomic origin of those positive clones, those sequences were compared to the nucleic acid database and protein database in GenBank by BLASTn and BLASTp. Most of those 9 sequences are homologous to the SVMPs family (sharing homology to reported gene with the highest identities of 85%). Among which, FII-2 and FII-4 shared the most conservative sequences, belonging to ADAM family, which have relationship with SVMPs family. So, we named the gene of FII-2 as FII und expressed the protein preferentially, and the amino acid sequences of FII was deduced (Fig.12).

### 6. Expression of F II gene from Agkistrodon acutus venom in E.Coli

Method: Added 0.2ml of E.Coli shaked overnight (colonies containing F II plasmid confirmed by extraction) to SOB medium containing 20ml of ampicillin shaked in 37°C for 3h until A₆₀₀ was up to about 0.5. Added IPTG to the final concentration of 1 umol/L. Shaked for 4h and centrifuged at 1,500 rpm for 20 min, and discarded the supernatant. Added 0.5ml of water and 0.5ml of SDS loading buffer to the pellet, heated in 100°C for 5min, and centrifuged at 10,000. 20ul of supernatant was taken to load for SDS-PAGE.

Result: Lysate of E.Coli containing FII plasmid after induced by IPTG showed a band of 40kD in SDS-PAGE, while those of E.Coli without IPTG induction or without plasmid did not showed such a band, indicating that the gene of FII can be expressed as fusion protein in F.Coli.

### 7. Cloning of FII gene of from Agkistrodon acutus venom

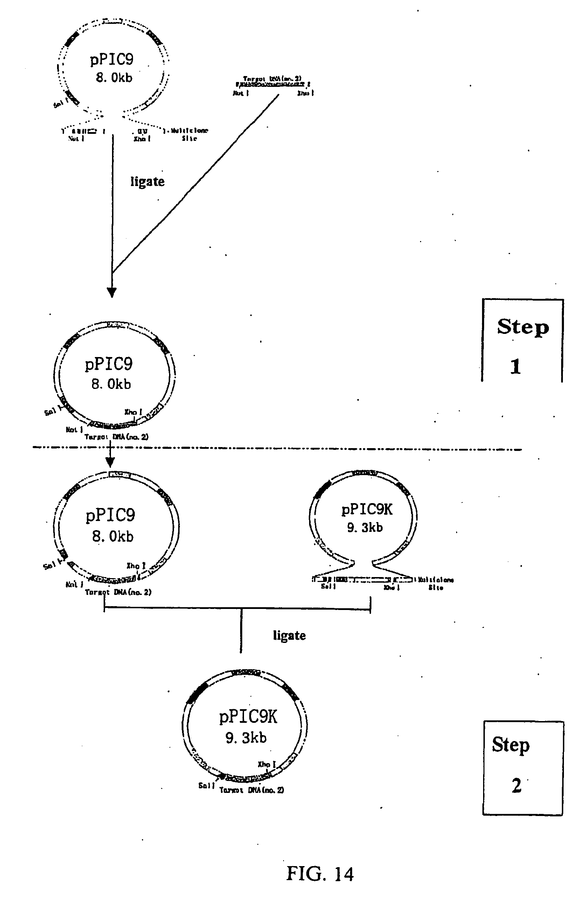

Method: (see fig.14)

### 7.1 PCR amplification of F II

The sequence of a signal to 5' of target gene was introduced by two-step PCR, and the restriction end nuclease site as well.
Primer 1:5' AGA GAG GCT GAA GCT AAT CTT ACT CCT GAA C 3'
Primer 2:5' CT CTC GAG AAA AGA GAG GCT GAA GCT AAT C 3'
Reverse primers:
Primer 3:5' GAG CGG CCG CCT CAC GCC TCC AAA AGT TC 3'
Procedure:
(1)The first round PCR: forward primer is primer 1, and reverse primer is primer 3; the template is F II plasmid. 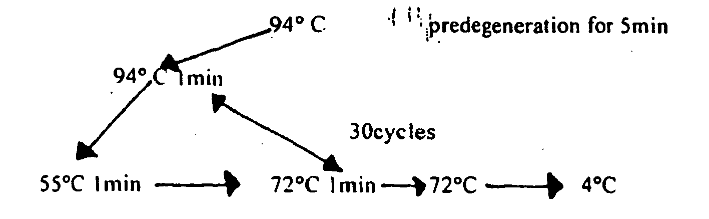
(2) The second round PCR: forward primer is primer 2, and reverse primer is primer 3; the template is the production of the first round of PCR. And the PCR condition is the same with the first round PCR.

### 7.2 Construction of recombinant PPIC9K-FII plasmid

The production of the second round PCR was extracted after electrophoresis on 1% agarose gel. The target fragment extracted from agarose gel and PPIC-9K plasmid were both digested with XhoI and NotI, and then retrieved after electrophoresis respectively; and then connected in T4 reaction system for 12h in 16°C. Then transformed to competent Top 10F cells and plated on I.B/penicillin plate. Picked up positive colonies, extracted plasmid and identified by restriction endonuclease analyses, and then purified the plasmid containing target fragment.

### 7.3 Construction of recombinant expression PPIC9K-F II plasmid

PPIC-9K plasmid with or without target fragment were digested both with Sacl and NotI, connected and transformed. And then picked up positive colonies, identified and purified PPIC-9K-FII plasmid.

Result: After the above two-step PCR, the PCR product wus about 700bp and was the same large with the gene of F II. The retrieved PCR product after electrophoresis and PPIC9K plasmid were digested both with XhoI and NotI, and then constructed recombinant PPIC9K-FII plasmid identified by restriction endonuclease digestion.

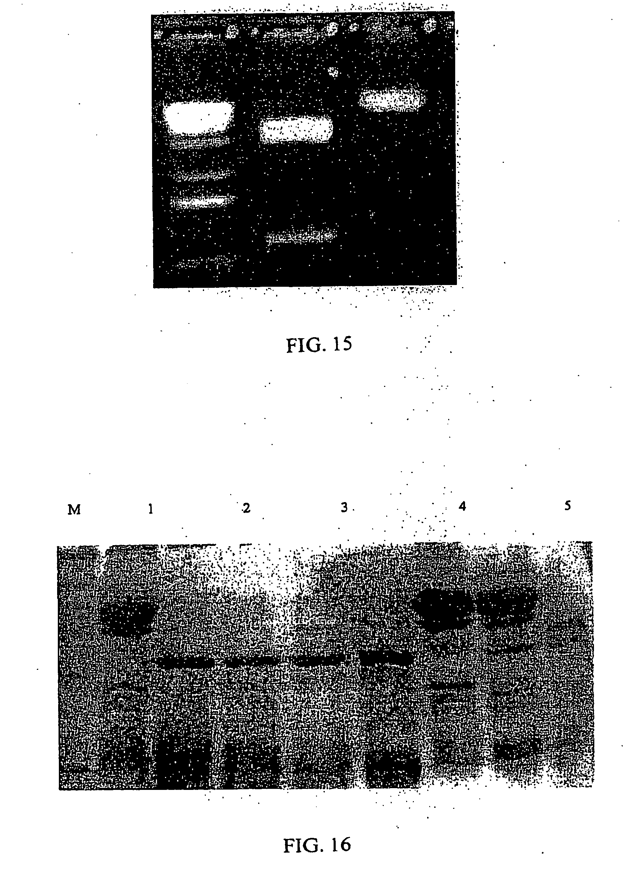

Digested recombinant PPIC9K-FII plasmid both with SucI and NotI. and subcloned the little digestion fragment to PPIC9K plasmid also digested both with SacI and Notl to construct recombinant expression PPIC9K-FII plasmid. The product identified by restriction endonuclease digestion was 700bp and was the same with anticipation (see fig.15). Sequencing by Sanger method showed that the DNA sequence of F II did exist in recombinant expression PPIC9K- FII plasmid.

### 8. Expression of the recombinant fibrinolytic enzyme FII from Agkistrodon acutus venom in yeast

### 8.1 Transformation and screening of yeast

Method: 50ug of recombinant plasmid PPIC9K-FII was isolated from the E.coli and linearized at the SacI restriction enzyme site, followed by extraction with phenol and chloroform, and precipitation with ethanol. After that, the DNA was dissolved in TE buffer and stored at -30°C. The linear plasmid mixed with the salmon sperm DNA and was added to competent yeast prepared with 3% polyglycol, which was plated to two RD plates well-distributedly. The plates grew at 30°C upside down. 96 hours later, the yeast strains were washed down the RD plates and plated on YPD plates containing a variety of G418 concentrations including 0.5, 1, 2, 3, 4 mg/ml. The YPD plates grew at 30°C. Recombinant colony first showed up in the plate with low G418 concentration on the forth day. On the 6th day, colonies from plate with 3mg/ml of G418 were selected to isolate DNA for PCR analysis.

Results: plasmid PPIC9K-FII was isolated and linearized at SacI site. The linear plasmid was purified and transformed into competent yeast with PEG method. The first colony showed up in YPD plate with G418 low concentration (0.5mg/ml) on day 4th, and on day 6th, 6 colonies were selected from the plate with 3mg/ml G418.

### 8.2 Screening for high level expression strain and scale-up expression of recombinant FII

Method: 6 recombinant strains which have been confirmed by PCR to contain our insert genes were inoculated in 10ml of MD media respectively, grew at 30°C overnight with shaking . Then harvest the cells by centrifugation, and added them into BMMY media to induce expression. Add 100% methanol to a final concentration of 1 % methanol every 24 hours to maintain induction. Sampling was also performed every day from the induction cultured and reacted with fibrinogen at 37°C, followed by SDS-PAGE, to analyze its fibrino(gen)lytic activity. 3 tubes of samples were quickly frozen on 3rd day.

Select a tube of frozen strain to 100ml MD media and cultivated at 30°C with shaking overnight and amplified to 2L culture in two 5 L flasks respectively. 24 hours later the cultures was transfered to BMMY media in jar fermenter of 100L volume and continued to grow at 30°C. And supplement with 1% methanol every 24h. After 96 hours fermentation, centrifuge the culture and saved the supernatant which was later ultrafiltrated into 2 L.

Results: 6 recombinant strains which have been confirmed to contain FII gene was cultivated. Sampling was done every day and its supernatant was used to react with fibrinogen at 37°C for 1 hour, and subjected to SDS-PAGE analysis. The 3rd day samples from number 1,2,3 yeast strains have showed hydrolysis activity towards fibrinogen (Fig. 16), natural FII was used as a positive control. The recombinant FII is identical with natural Fll in terms of hydrolysis modality and dominant fraction after hydrolysis, which suggested that these three yeast strains are capable of expressing recombinant FII in high efficiency. Large scale recombinant Fll can be obtained in culture media supernatant after 96 hours fermentation with methanol induction.

### 9. Purification and biochemical characterization determination of the recombinant fibrinolytic enzyme F II from Agkistrodon acutus venom

### 9.1 DEAE-Sepharose FF negative ion exchange chromatography

Method:
(1) ultra filtration. The supernatant liquor was ultra filtrated by Millipore until the column was 2L, added double column of 0.05M NH4Ac and continued to ultra filtrate.
(2) DEAE-Sepharose FF. The supernantant ultrafiltered was applied to a column of DEAE-Sepharose FF previously equilibrated with 0.05M NH4Ac (PH8.0). The column was washed with 0.1 M NH4Ac (PH6.5) to remove unbound material and the remained proteins were then eluted with 0.2M NII4Ac (PH5.2).

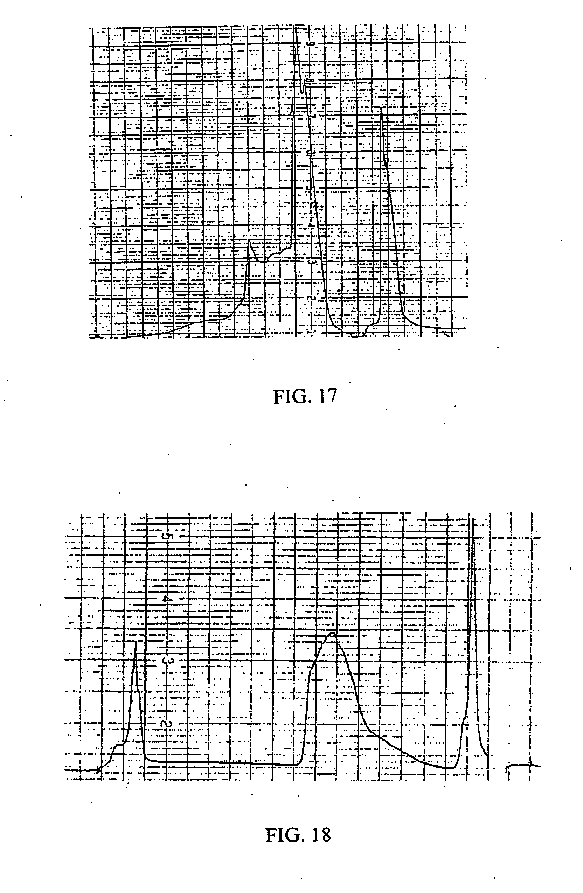

Results: The supernantant ultrafiltered on DEAE-sepharose yielded 5 fractions (Fig17). The fibrinolytic activity was localized in the fraction in the 0.2M NH4Ac (PH5.2).

### 9.2 Hydrophobic chromatography

Method: The fraction displaying fibrinogenolytic activity was then mixed 2M NaCl and applied to another column of Butyl-toyopearl 4 FF previous equilibrated 1M NaCl, 20mM PBS. The column was washed with 1M NaCl, 20mM PBS to remove unbound material and the remained proteins were then eluted with 2mM PBS.

Results: The supernantant ultrafiltered of the fraction on Butyl-toyopearl gave 3 fractions (Fig 18). The fibrinolytic activity was localized in the third fraction recombant F II was obtained by ultrafiltering, purifying from salts and lyophilizing the fraction displaying fibrinogenolytic activity.

### 9.3 Purity determination of the recombinant fibrinolytic Enzyme FII from Agkistrodon acutus venom

Method: The Purity determination of recombinant FII was performed by the method of SDS-PAGE mentioned as decribe in methods 2.1.

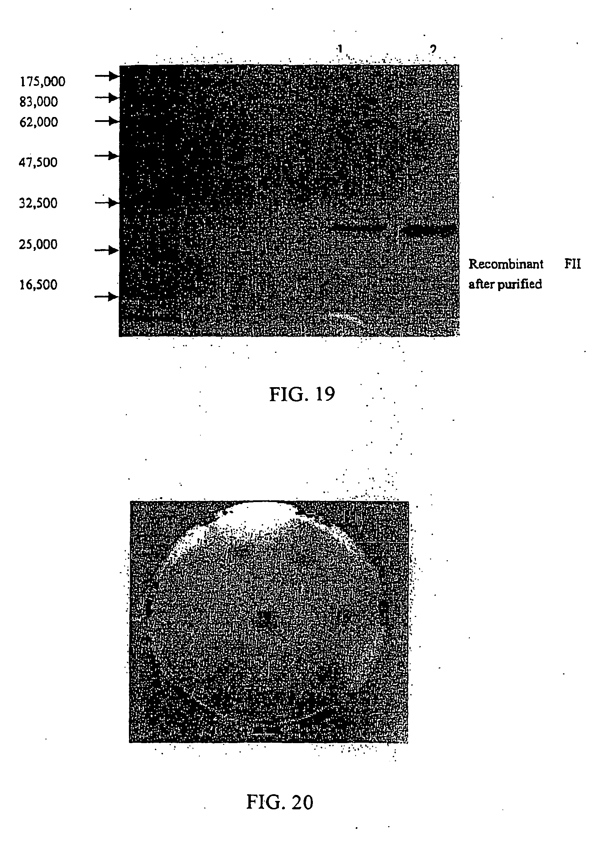

Results: On the SDS-PAGE gel, the purified recombinat FII appeared as a single protein band (Fig 19).

### 9.4 The directly fibrinolytic activity determination of the recombinant fibrinolytic enzyme FII from Agkistrodon acutus venom

Method: The fibrinolytic activity of FII was demonstrated using a heated fibrin plate which was incubated at 85°C for 30min in order to inactivate the plasmin activity. 20 microliters of different concentration of recombinant FII (2mg/ml, 1mg/ml, 0.5mg/ml, 0.25 mg/ml, 0.125 mg/ml), were applied onto the prepared plate surface. Nature FII 0.5mg/ml and urokinase 500U/ml were used for positive control and the saline solution was used for the negative control. Each concentration was tested 3 times. The plate was incubated in a humid chamber at 37°C for 12h. Fibrinolytic activity was measured by the area of the clear lysed zone.

Results: The fibrinolytic activity of FII was demonstrated using a heated fibrin plate technique. It was shown that rF II could formed clear lysis zone on the fibrin plate with increasing concentration, its fibrinolytic activity was higher than nature F II (the lysed zone by 0.5mg/ml rF II was 50mm², and the lysed zone by 0.5mg/ml nature FII was only 40mm²) ( table 2, Fig20). The plasmin was inactivated when the fibrin plate was heated to 85°C; it was showed that recombinant FII could dissolve fibrin without depending on the plasminogen activation.

### 9.5 The effect of recombinant F II on human IgG and albumin.

Method: Human IgG(10µl, 10mg/ml) mixed with r-F II (10µl, 0.5mg/ml) was incubated at 37°C for 24h using a pipette. After the indicated time, 10µl Protein electrophoresis Solution was added. The sample was analyzed by SDS-PAGE. The same experiment was repeated except for human albumin in place of human IgG.

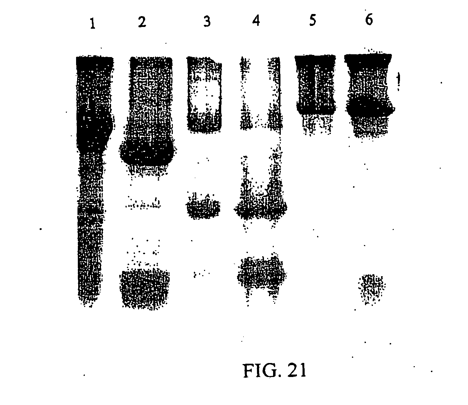

Results: When IgG was incubated with recombinant FII, the Heavy Chain disappeared, while the light chain still insusceptible to the enzyme. When albumin was incubated with recombinant FII, the peptide chains almost remained integrity. It suggested that recombinant FII have no effect on albumin (Fig21).

### 10. The effect of recombinant FII un the Beagle's Pulmonary Artery thrombosis.

Method: Beagle's Pulmonary Artery thrombosis experimental models were performed by the method of Nowk. The blood clot was prepared by incubating the 15ml experimental Beagle's whole blood at 37°C for 2h in vitro before application, Two different groups were established, containing 6 animals each : the treatment group was given 0.24 mg/kg r FII. The NS control group was infused with 0.1ml/kg NS. Animals were anaesthetized by an intramuscular injection of 30 mg/kg pentobarbital sodium, followed by intramuscular supplements of ketamine hydrochloride given throughout the experiment. The left femoral vein was separated from surrounding tissue after anesthesia. A 6-mm² Fcohra/Humterhead catheter sheath was drown into the left femoral vein, through Infrarenal abdominal vein, Right atrial, Right ventricular, Pulmonary trunk , and was finally introduced in the right lower Pulmonary artery. The artificial thrombus was injected into the catheter positioned in the right lower Pulmonary artery. Selective intra-arterial digital subtraction angiography (DSA) was performed on a biplane high-resolution angiography system with a matrix of 1024*1024 pixels to record the thromboembolism in the right lower Pulmonary artery. Two h ours after the infusion of the preformed thrombus, single dosages of recombinant F II were injected through the femoral vein. The recanalization rate of thrombosis of the right lower Pulmonary artery was measured by the lateral and auteroposterior(AP) projection images before rF II infusion and at 15,30,60,90 and 120 mins post-infusion to evaluate the effect of r FII on Artery thrombosis.

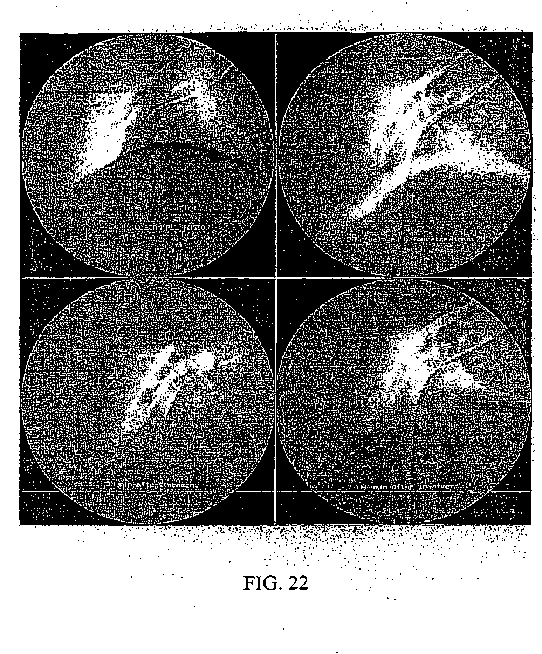

Results: Measurements made at 1 hours post recombinant F II infusion showed that recombinant F II efficiently dissolve the preformed thrombus in the right lower Pulmonary artery (Fig22). The average of the recanalization rate of thrombosis in the right lower Pulmonary artery was 83.3%. Little changes were detected in the NS control groups measured 1 hours post infusion. The average of the recanalization rates of thrombosis in the right lower Pulmonary artery was 0.6% (table 3).

## Claims

1. An isolated polynucleotide consisting of the following sequence:

5' -aaaagagaggctgaagctaatcgtactcctgaacaacaaatctatgacccctacaaatacgttga
gactgtctttgttgtggacaaagcaatggtcacaaaatacaatggcgatttagataagataaaaacaag
aatgtacgaagctgccaacaatatgaatgagatgtacagatatatgtttttcgtgtagtaatggttgg
cctaataatttggaccgaagaagataagattaccgtgaagccagatgtggattatactttgaacgcatt
tgcagaatggagaaaaacatatttgctggctgagaaaaacatgataatgctcagttaatcacgggcat
tgacttcagaggaagcattataggatacgcttacattggcagcatgtgccacccgaagcgttctgtagg
aattattcaggattatagcccaataaatcttgtgcttgccgttataatggcccatgagatgggtcacaa
tctgggcattcaccatgacgacggttactgttattgcggtggttacccatgcattatgggtccctcgat
aagccctgaaccttccaaatttttcagcaattgtagttatatccaatgttgggactttattatgaatca
caacccagaatgcattgacaatgaacccttgggaacagatattatttcacctccactttgtggaaatga
actttggaggcgtga-3' (702bp).

2. A DNA vector containing the polynucleotide according to claim 1.

3. A host cell containing the DNA vector according to claim 2.

4. A recombinant fibrinolytic enzyme produced by the host cell according to claim 3 and consisting of the amino acid sequence

XKREAEANRTPEQQIYDPYKYVETVFVVDKAMVTKYN
GDLDKIKTRMYEAANNMNEMYRYMFFRVVMVGLIIW
TEEDKITVKPDVDYTLNAFAEWRKTYLLAEKKHDNAQ
LITGIDFRGSIIGYAYIGSMCHPKRSVGIIQDYSPINLVLA
VIMAHEMGHNLGIHHDDGYCYCGGYPCIMGPSISPEPS
KFFSNCSYIQCWDFIMNHNPECIDNEPLGTDIISPPLCGN
ELLEA

5. A pharmaceutical composition including the recombinant fibrinolytic enzyme according to claim 4.

6. The recombinant fibrinolytic enzyme according to claim 4 for use against thromboembolic disorders.

## Patentansprüche

1. Isoliertes Polynukleotid, das aus der folgenden Sequenz besteht:

5' -aaaagagaggctgaagctaatcgtactcctgaacaacaaatctatgacccctacaaatacgttga
gactgtctttgttgtggacaaagcaatggtcacaaaatacaatggcgatttagataagataaaacaag
aatgtacgaagctgccaacaatatgaatgagatgtacagatatatgtttttcgtgtagtaatggttgg
cctaataatttggaccgaagaagataagattaccgtgaagccagatgtggattatactttgaacgcatt
tgcagaatggagaaaaacatatttgctggctgagaaaaacatgataatgctcagttaatcacgggcat
tgacttcagaggaagcattataggatacgcttacattggcagcatgtgccacccgaagcgttctgtagg
aattattcaggattatagcccaataaatcttgtgcttgccgttataatggcccatgagatgggtcacaa
tctgggcattcaccatgacgacggttactgttattgcggtggttacccatgcattatgggtccctcgat
aagccctgaaccttccaaatttttcagcaattgtagttatatccaatgttgggactttattatgaatca
caacccagaatgcattgacaatgaacccttgggaacagatattatttcacctccactttgtggaaatga
actttggaggcgtga-3' (702bp).

2. DNA-Vektor, der das Polynukleotid gemäß Anspruch 1 enthält.

3. Wirtszelle, die den DNA-Vektor gemäß Anspruch 2 enthält.

4. Rekombinantes fibrinolytisches Enzym, das durch die Wirtszelle gemäß Anspruch 3 erzeugt worden ist und aus der Aminosäuresequenz 
XKREAEANRTPEQQIYDPYKYVETVFVVDKAMVTKYN
GDLDKIKTRMYEAANNMNEMYRYMFFRVVMVGLIIW
TEEDKITVKPDVDYTLNAFAEWRKTYLLAEKKHDNAQ
LITGIDFRGSIIGYAYIGSMCHPKRSVGIIQDYSPINLVLA
VIMAHEMGHNLGIHHDDGYCYCGGYPCIMGPSISPEPS
KFFSNCSYIQCWDFIMNHNPECIDNEPLGTDIISPPLCGN
ELLEA
 besteht.

5. Pharmazeutische Zusammensetzung, die das rekombinante fibrinolytische Enzym gemäß Anspruch 4 umfasst.

6. Rekombinantes fibrinolytisches Enzym gemäß Anspruch 4 zur Verwendung gegen thromboembolische Störungen.

## Revendications

1. Polynucléotide isolé constitué de la séquence suivante :

5' -aaaagagaggctgaagctaatcgtactcctgaacaacaaatctatgacccctacaaatacgttga
gactgtctttgttgtggacaaagcaatggtcacaaaatacaatggcgatttagataagataaaaacaag
aatgtacgaagctgccaacaatatgaatgagatgtacagatatatgtttttcgtgtagtaatggttgg
cctaataatttggaccgaagaagataagattaccgtgaagccagatgtggattatactttgaacgcatt
tgcagaatggagaaaaacatatttgctggctgagaaaaacatgataatgctcagttaatcacgggcat
tgacttcagaggaagcattataggatacgcttacattggcagcatgtgccacccgaagcgttctgtagg
aattattcaggattatagcccaataaatcttgtgcttgccgttataatggcccatgagatgggtcacaa
tctgggcattcaccatgacgacggttactgttattgcggtggttacccatgcattatgggtccctcgat
aagccctgaaccttccaaatttttcagcaattgtagttatatccaatgttgggactttattatgaatca
caacccagaatgcattgacaatgaacccttgggaacagatattatttcacctccactttgtggaaatga
acttttggaggcgtga-3' (702bp).

2. Vecteur d'ADN contenant le polynucléotide selon la revendication 1.

3. Cellule hôte contenant le vecteur d'ADN selon la revendication 2.

4. Enzyme fibrinolytique recombinante produite par la cellule hôte selon la revendication 3, et constituée de la séquence d'acides aminés suivante :

XKREAEANRTPEQQIYDPYKYVETVFVVDKAMVTKYN
GDLDKIKTRMYEAANNMNEMYRYMFFRVVMVGLIIW
TEEDKITVKPDVDYTLNAFAEWRKTYLLAEKKHDNAQ
LITGIDFRGSIIGYAYIGSMCHPKRSVGIIQDYSPINLVLA
VIMAHEMGHNLGIHHDDGYCYCGGYPCIMGPSISPEPS
KFFSNCSYIQCWDFIMNHNPECIDNEPLGTDIISPPLCGN
ELLEA

5. Composition pharmaceutique comprenant l'enzyme fibrinolytique recombinante selon la revendication 4.

6. Enzyme fibrinolytique recombinante selon la revendication 4, pour une utilisation contre des troubles thromboemboliques.
